# EUROPÄISCHE PATENTSCHRIFT

(11) **EP 1 694 231 B1**
(45) Veröffentlichungstag und Bekanntmachung des Hinweises auf die Patenterteilung: **26.09.2007**
(21) Anmeldenummer: 04744236.3
(22) Anmeldetag: 11.08.2004
(51) Int. Cl.: A61B 17/92, B25D 1/16

(54) **CHIRURGISCHER HAMMER**
SURGICAL MALLET
MAILLET CHIRURGICAL

(30) Priorität: 08.12.2003 CH 208303
(43) Veröffentlichungstag der Anmeldung: 30.08.2006
(73) Patentinhaber: Synthes GmbH, 4436 Oberdorf (CH)
(72) Erfinder: BÜTTLER, Markus, CH-4702 Oensingen (CH); STREFF, Patrick, 79576 Weil am Rhein (DE)
(74) Vertreter: Rosenich, Paul
(86) Internationale Anmeldenummer: PCT/IB2004/002601
(87) Internationale Veröffentlichungsnummer: WO 2005/055845

(56) Entgegenhaltungen:
- US-A- 5 906 210
- US-B2- 6 592 590

## Beschreibung

Die Erfindung betrifft einen chirurgischen Hammer, der bei der Insertion und/oder Extraktion von implantaten, insbesondere intermedullären Nägeln und Kirschner-Drähten, aber auch für sonstige Hammeranwendungen in der Chirurgie vorgesehen ist.

Aus der US-A-5,476,467 und der WO-A1-80/00534 sind chirurgische Hämmer bekannt, die auf Vorrichtungen für das Inserieren und/oder Extrahieren von Implantaten, insbesondere intermedullären Nägeln, geführt sind. Diese Hämmer haben die Form eines Zylinders und sind mit einer zentrierten, der Längsachse folgenden Bohrung versehen, mit welcher der Hammer über die Insertions- bzw. Extraktionsvorrichtung gleitet. Nachteilig an diesen Hämmern ist, dass eine Führung des Hammers schwierig ist, da der Chirurg nur Kraft auf den Hammer ausüben kann, indem er mit seiner Hand den Zylinder auf dessen Mantelfläche umgreift. Weiterhin ist nachteilig, dass diese Hämmer nur mit der jeweiligen Einführvorrichtung verwendet werden können.

Verbesserte chirurgische Hämmer sind aus der US-A-5,913,860 und aus der DE-C1-198 60 569 bekannt, die ähnlich den zuvor zitierten Dokumenten Hämmer mit zylindrischem Hammerkopf offenbaren, die jedoch zusätzlich einen Hammerstiel aufweisen, über den die Führung der chirurgischen Hämmer erleichtert wird. Ferner weist der chirurgische Hammer der US-A-5,913,860 nicht nur eine zylindrische Bohrung auf, sondern eine Ausnehmung, so dass der chirurgische Hammer nach dem Zusammenbauen der Einführvorrichtung eingeführt werden kann. Weiterhin ist dadurch dieser chirurgische Hammer auch universeller einsetzbar; so kann er auch als Hammer zum unmittelbaren Eintreiben von beispielsweise Nägeln verwendet werden. Nachteilig ist jedoch, dass die Führung des Hammers auf der Einführvorrichtung insofern erschwert ist, als durch die geschlitzte Gestaltung der Hammerkopf nicht mehr sicher auf der Implantationsvorrichtung, beispielsweise der Führungsstange gehaltert ist, sondern der Chirurg beim Bewegen des Hammers darauf achten muss, dass er nicht von der Führungsstange abrutscht. Dadurch wird zum einen die Präzision verschlechtert und zum anderen steigt die Verletzungsgefahr für den Operateur bzw. das ihn umgebende Personal.

Ein weiterer Nachteil ist, dass die bei Hämmern üblicherweise zum Schlagen verwendete Grundfläche des zylindrischen Hammerkopfes geschlitzt ist. Dadurch ist die Handhabung beim Schlagen erschwert, da darauf geachtet werden muss, dass nicht mit dem Schlitz der Nagelkopf bzw. das Nagelende oder sonstige Instrumente, die für das Inserieren verwendet werden, getroffen werden, sondern mit der unversehrten Fläche. Deshalb werden derartig gestaltete Hämmer üblicherweise als Schlaginstrument verwendet, indem die Mantelfläche als solche verwendet wird. Bei der geschlitzten Gestaltung des Hammerkopfes bringt dies jedoch den Nachteil, dass der Hammerkopf beim Aufschlag dazu neigt, zu federn.

Aus der US-B2-6,592,590 ist ein chirurgischer Hammer bekannt, der einen Führungskanal für eine Führungsstange aufweist, der so gestaltet ist, dass der Hammerkopf davon abgehalten wird, von der Führungsstange in lateraler Richtung entfernt zu werden. Nachteilig an dieser Vorrichtung ist jedoch, dass die Schlagfläche des Hammerkopfes ebenfalls durchbrochen ist, so dass der Hammer nur in Verbindung mit einer Führungsstange zuverlässig verwendet werden kann. Ferner ist die Führung im Vergleich zu geschlitzten Hämmern verbessert, dennoch neigt der dort offenbarte Hammerkopf bereits bei leichtem Verkanten dazu, von der Führungsstange herunter zu rutschen. Dies passiert vergleichsweise schnell, da es der natürlichen Bewegungsrichtung des Menschen zuwider läuft, eine exakt vertikale Schlagbewegung auszuführen. Günstiger ist es, einen leichten Bogen beim Schlag auszuführen, genau dies führt jedoch dazu, dass die Führungsstange aus der Führung im Hammerkopf gleitet.

Der Erfinder erkannte folglich, dass die bekannten Systeme nachteilig sind, insbesondere in Bezug auf die folgenden Punkte:
a) die Führung über die Vorrichtung zum Inserieren und/oder Extrahieren der chirurgischen Implantate, beispielsweise über eine Führungsstange; und
b) die Verwendung als Schlaginstrument, wegen der federnden Gestaltung des geschlitzten chirurgischen Hammers bzw. der durchbrochenen Schlagfläche.

Der Erfindung liegt somit die Aufgabe zugrunde, einen chirurgischen Hammer bereitzustellen, welcher der Vorrichtung zum Inserieren und/oder Extrahieren der Implantate eine gute Führung bereitstellt, nach dem Zusammenbau in diese eingesetzt werden kann, und mit dem vorzugsweise auch ausserhalb der Vorrichtung zum Inserieren und/oder Extrahieren zuverlässig geschlagen werden kann. Er soll somit die angegebenen Nachteile vermeiden und noch universeller und schlagsicherer als bisherige Hämmer einsetzbar sein.

Gelöst wird diese Aufgabe durch einen chirurgischen Hammer nach Anspruch 1. Vorteilhafte Ausgestaltungen sind Gegenstand der Unteransprüche.

Der chirurgische Hammer gemäss der Erfindung weist einen Hammerkopf auf, wobei der Hammerkopf eine im Wesentlichen zylindrische Gestalt aufweist. Er ist mit zumindest einer Ausnehmung versehen, in der eine Vorrichtung zum Inserieren und/oder Extrahieren von Implantaten, insbesondere von intermedullären Nägeln und Kirschner-Drähten, z. B. eine Führungsstange, auch als Extraktionsstange oder Gleitstange bezeichnet, aufnehmbar ist. Erfindungsgemäss ist die Ausnehmung durch mindestens zwei gesonderte Bereiche gebildet, nämlich durch Einführkanal und Verriegelungsraum, wobei die Vorrichtung zum Inserieren und/oder Extrahieren im Verriegelungsraum verriegelbar ist. Weiter ist erfindungsgemäß die Grundfläche des Hammerkopfes in sich geschlossen. Gemäss einem bevorzugten Ausführungsbeispiel erstreckt sich der Verriegelungsraum im Wesentlichen in radialer Richtung.

Der Bereich, in den die Vorrichtung zum Inserieren und/oder Extrahieren zuerst eingeführt wird, wird nachstehend als Einführkanal bezeichnet, und der Bereich, in dem die Vorrichtung nach dem Einführen zum Schlagen geführt wird, wird nachstehend als Verriegelungsraum bezeichnet.

Unter den Begriff "Vorrichtung zum Inserieren und/oder Extrahieren von Implantaten" fallen alle Vorrichtungen, die für die Insertion und/oder Extraktion eines Implantates, z. B. eines Marknagels oder Kirschner-Drahtes, verwendbar sind. Insbesondere wird für die vorliegende Erfindung darunter eine Führungsstange verstanden, die auch als Extraktionsstange oder Gleitstange bezeichnet wird.

Die Ausnehmung und damit der Einführkanal und der Verriegelungsraum stellen Führungsflächen für die Führung der Vorrichtung zum Inserieren und/oder Extrahieren, z. B. der Führungsstange bereit. Die Vorrichtung zum Inserieren und/oder Extrahieren wird in der Ausnehmung nicht nur geführt, sondern es erfolgt im Verriegelungsraum eine Verrieglung der Stange, so dass sich diese während des Insertions- bzw. Extraktionsvorganges nicht von dem Hammerkopf lösen kann. Die Anordnung der Bestandteile der Ausnehmung verhindert das Ausfahren der Vorrichtung zum Inserieren und/oder Extrahieren während dem Hämmern. Selbst wenn der Hammer etwas verkantet angetrieben würde, kann der Hammerkopf nicht von der Führungsstange abrutschen. Vorteilhaft ist weiter, dass sich für den Chirurgen eine angenehmere Schlagrichtung ergibt, da im Verriegelungsraum ein gewisses Spiel verbleibt, so dass auch ein leichter Bogen beim Schlagen ausgeführt werden kann und die Führungsstange trotzdem noch eine sichere Führung erfährt.

Da die Grundfläche des Hammerkopfes in sich geschlossen ist, kann der Hammer als "normaler" Hammer zum Schlagen genutzt werden. Es sind folglich, wie von einem Hammer allgemein gewohnt, zwei Schlagflächen vorhanden.

Gemäss einem ersten bevorzugten Ausführungsbeispiel ist die Ausnehmung durch drei gesonderte Bereiche, nämlich Einführkanal, Axialkanal und Verriegelungsraum gebildet, die miteinander in Verbindung stehen. Die Bereiche sind insoweit gesondert, dass jeder Bereich für sich alleine einen Raum innerhalb des Hammers beansprucht, der nicht deckend mit einem weiteren Raum oder Kanal ist. Jeder Kanal und auch der Verriegelungsraum bildet einen Führungsbereich für eine Vorrichtung zum Inserieren und/oder Extrahieren von Implantaten, der von der Vorrichtung seines gesamten Umfangs nach durchschritten werden muss, bevor in den nächsten, sich anschließenden, Kanal oder Raum zu gelangen ist. Vorteilhaft erfährt die Vorrichtung zum Inserieren und/oder Extrahieren, insbesondere eine Führungsstange, dadurch eine im Vergleich zu den Vorrichtungen des Standes der Technik verbesserte Führung.

Die Ausnehmung ist, anders ausgedrückt, so gestaltet, dass die Vorrichtung zum Inserieren und/oder Extrahieren bajonettverschlussartig einführbar ist. Die Vorrichtung wird folglich in den Hammer eingefädelt, wobei jedoch dies nicht, wie bei den im Stand der Technik bekannten geschlitzten Hämmern, durch eine einzelne horizontale bzw. vertikale Bewegung möglich ist, oder wie bei dem aus der US-B2-6,592,590 bekannten Hammer durch eine horizontale und eine Kippbewegung, sondern die Ausnehmung ist so gestaltet, dass mindestens eine überwiegend horizontale und mindestens eine überwiegend vertikale Einführbewegung benötigt wird, wobei zusätzlich noch eine Drehbewegung ausgeübt werden muss.

Die Ausnehmung ist somit winkelig gestaltet, wobei der Bereich, in den die Vorrichtung zuerst eingeführt wird, nachstehend als Einführkanal bezeichnet, und der Bereich, in dem die Vorrichtung nach dem Einführen zum Schlagen geführt wird, nachstehend als Verriegelungsraum bezeichnet wird. Die Achsen dieser Räume verlaufen nicht parallel, sondern sie sind zueinander winkelig angeordnet.

Bevorzugt ist ferner, dass sich Einführkanal und Verriegelungsraum nicht direkt schneiden. Im Gegenteil, ihre Ebenen verlaufen in bevorzugten Ausführungsbeispielen annähernd parallel. Einführkanal und Verriegelungsraum sind statt dessen durch den Axialkanal verbunden.

Gemäss einem weiteren Ausführungsbeispiel der Erfindung ist der Einführkanal in einem Winkel von annähernd ca. 20-70°, insbesondere von ca. 40-50° zu der Längsachse des Hammerkopfes angeordnet. Der Einführkanal liegt folglich schräg in der Mantelfläche des Hammerkopfes. Einführkanal und Verriegelungsraum sind zueinander in einem Winkel von annähernd ca. 20-70°, insbesondere von ca. 30-40° zueinander angeordnet. Der Verriegelungsraum ist folglich bevorzugt radial ausgerichtet, wie oben beschrieben. Beim Einführen der Vorrichtung zum Inserieren und/oder Extrahieren wird der Hammerkopf folglich schräg auf diese aufgesetzt und in den Einführkanal eingeschoben. Anschließend wird der Kopf in die horizontale Position gedreht und weiter in den Verriegelungsraum eingeschoben. Dadurch ist die Vorrichtung zum Inserieren und/oder Extrahieren in dem Verriegelungsraum verriegelt und der Hammer kann zum Schlagen für die Insertion oder Extraktion verwendet werden. Auch bei diesem Ausführungsbeispiel verhindert die Anordnung der Bestandteile der Ausnehmung das Ausfahren der Vorrichtung zum Inserieren und/oder Extrahieren während dem Hämmern. Selbst wenn der Hammer etwas verkantet angetrieben würde, kann der Hammerkopf nicht von der Führungsstange abrutschen. Vorteilhaft ist weiter, dass sich für den Chirurgen eine angenehmere Schlagrichtung ergibt, da im Verriegelungsraum ein gewisses Spiel verbleibt, so dass auch ein leichter Bogen beim Schlagen ausgeführt werden kann und die Führungsstange trotzdem noch eine sichere Führung erfährt.

Gemäss einem bevorzugten Ausführungsbeispiel wird der Verriegelungsraum durch versetzt angeordnete Vorsprünge begrenzt. Diese Vorsprünge verhindern zusätzlich zu der erfindungsgemäßen Anordnung der Einzelbereiche der Ausnehmung das Abrutschen des Hammerkopfes von der Vorrichtung zum Inserieren und/oder Extrahieren. Letztere wird durch die Vorsprünge zusätzlich zurückgehalten.

Weitere Ausbildungen der Erfindung und Varianten dazu sind in den abhängigen Patentansprüchen sowie in der Figurenbeschreibung angegeben.

Im Sinne der Erfindung liegen auch Hämmer mit einer nicht zylinderförmigen Ausgestaltung. So könnte die Mantelfläche auch tonnenförmig ausgebildet sein oder zylindrische oder flache Schlagflächen umfassen, während der Rest zu den Grundflächen hin tonnenförmig verläuft.

Durch die erfindungsgemässe Gestaltung der Ausnehmung im Hammerkopf werden insbesondere die folgenden Verbesserungen erzielt:
■ Obwohl der Hammerkopf nach dem Zusammenbau der Vorrichtung zum Inserieren und/oder Extrahieren eingeführt werden kann, wird eine Art Verriegelung des Hammerkopfes über der Vorrichtung erzielt. Durch das Vorsehen von gesonderten Bereichen, nämlich Einführkanal, Axialkanal und Verriegelungsraum, ist das Einführen der Führungsstange so komplex, dass es nur durch dieselbe bajonettverschlussartige Führung in entgegengesetzter Richtung möglich ist, den Hammerkopf wieder von der Vorrichtung abzunehmen. Eine derartige Bewegung wird jedoch beim Schlagen mit dem Hammer nicht durchgeführt, folglich bleibt der Hammerkopf auch während des Schlagens über der Vorrichtung verriegelt.
■ Durch die sichere Führung sind präzise Schläge möglich, was sich sowohl beim Eintreiben als auch beim Austreiben von Implantaten positiv bemerkbar macht, da Verletzungen und Beschädigungen von Implantaten und Instrumenten oft durch unpräzise Schläge zustande kommen.
■ Durch die erfindungsgemässe Gestaltung der Ausnehmung im Hammerkopf bleibt die volle Schlagkraft erhalten. In der bevorzugten Ausführungsform ist die Ausnehmung ausschliesslich in der Mantelfläche vorgesehen; die Grundflächen bleiben somit grundsätzlich unversehrt.
■ Dadurch bleibt die gesamten Grundflächen des chirurgischen Hammers als Schlagflächen erhalten. Selbst wenn jedoch die Ausnehmung so angelegt ist, dass auch die Grundflächen betroffen wären, bleibt jedoch die Schlagkraft über die Mantelflächen weitgehend erhalten, da durch die bajonettverschlussartige Anordnung von Einführkanal, Axialkanal und Verriegelungsraum der Ausnehmung ein Federn beim Schlagen überwiegend verhindert wird.
■ Wenn zudem der Hammerkopfschwerpunkt in die verlängerte Hammerstielachse und/oder - im eingefädelten Zustand - in die Achse einer Führungsstange gelegt ist, dann ist zudem die Gefahr des Verkantens während dem Gleiten an der Führungsstange minimiert.

Anhand von Zeichnungen wird die Erfindung noch weiter beispielhaft und nicht einschränkend erläutert. Die Figuren werden übergreifend beschrieben. Gleiche Elemente tragen gleiche Bezugszeichen. Elemente mit ähnlichen Aufgaben, jedoch unterschiedlicher Ausbildung, tragen gleiche Bezugszeichen mit unterschiedlichen Indizes. Die Figurenbeschreibung, Bezugszeichenliste und Patentansprüche ergänzen einander im Sinne der Offenbarung der Erfindung.

Es zeigen dabei:
- Fig. 1: eine schematische Darstellung eines erfindungsgemässen chirurgischen Hammers in seiner erfindungsgemässen Verwendung;
- Fig. 2: eine schematische Darstellung der Bewegungsführung der Vorrichtung zum Inserieren und/oder Extrahieren in der Ausnehmung des erfindungsgemässen Hammers;
- Fig. 3: ein bevorzugtes Ausführungsbeispiel eines chirurgischen Hammers in unterschiedlichen Ansichten;
- Fig. 4: den Hammerkopf des chirurgischen Hammers in verschiedenen Ansichten;
- Fig. 5: eine perspektivische Darstellung in Vorderansicht eines erfindungsgemässen chirurgischen Hammers;
- Fig. 6: den Hammer der Fig. 5 in Rückansicht;
- Fig. 7: ein weiteres Ausführungsbeispiel eines erfindungsgemässen chirurgischen Hammers in Aufsicht (A) und Seitenansicht (B);
- Fig. 8: den Hammerkopf des Hammers der Fig. 7 in verschiedenen Ansichten; und
- Fig. 9: die Funktionsweise des Hammerkopfes der Fig. 7 und 8.

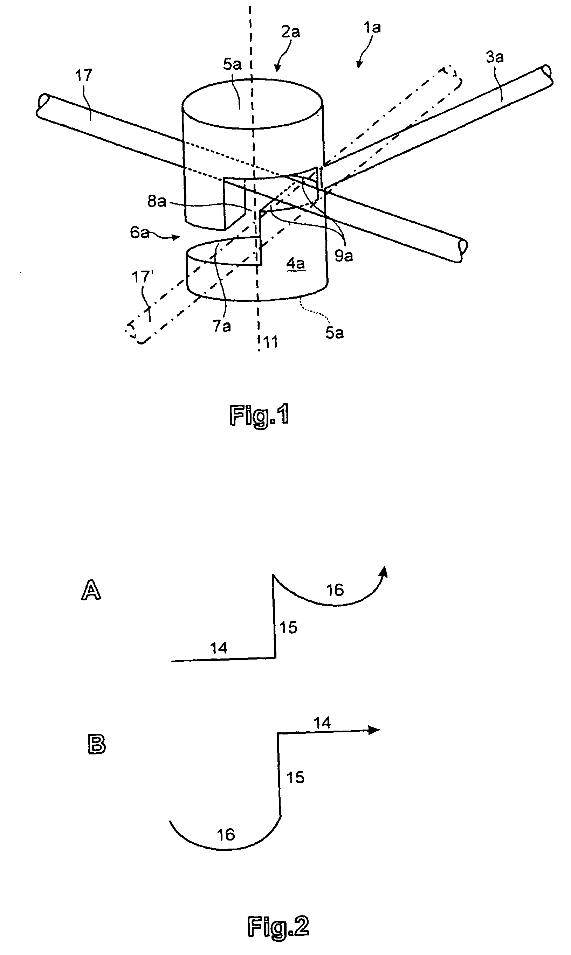

In Fig. 1 ist schematisch ein chirurgischer Hammer an einer Führungsstange 17 gemäss der Erfindung dargestellt. Der chirurgische Hammer 1a weist einen Hammerkopf 2a und einen Hammerstiel 3a auf. Der Hammerstiel 3a ist nur ansatzweise dargestellt. In den Hammer 1 a ist die Führungsstange 17 eingeführt, wobei die Position der Führungsstange 17 unmittelbar nach dem Einführen in die Ausnehmung 6a durch die strichpunktierte Führungsstange 17' angedeutet ist. Die Endposition der Führungsstange 17, das heisst die Position, in welcher der Hammer 1a über der Führungsstange geführt wird, und in welcher die Schlagkraft mit dem Hammer ausgeübt wird, ist durch die durchgezogene Linie der Führungsstange 17 dargestellt. Auch die Führungsstange 17 ist - da sie herkömmlich ist - nicht in ihrer gesamten Ausgestaltung und Länge dargestellt.
Der Hammerkopf 2a weist eine Mantelfläche 4a und zwei Grundflächen 5a auf. Im Hammerkopf 2a ist eine Ausnehmung 6a ausgebildet, welche einen Einführkanal 7a, einen Axialkanal 8a und einen Verriegelungsraum 9a aufweist. Die Ausnehmung 6a ist schlitzartig gestaltet. Die Grösse des Schlitzes richtet sich nach der einzuführenden Vorrichtung, dies bedeutet, dass der Durchmesser der Ausnehmung 6a an den Durchmesser der Führungsstange 17 angepasst ist. Jedenfalls ist die Ausnehmung 6a in radiale Richtung - in Bezug auf die Längsachse 11 des Hammerkopfes - wesentlich kürzer, als herkömmliche Schlitze in herkömmlichen geschlitzten Hämmern; jedoch etwa gleich lang, wie bei dem Hammer gemäss US-B2-6592590, wobei im Unterschied dazu der erfindungsgemässe Einführkanal 7a aussermittig angeordnet ist, so dass die im grösseren Abstand zur Ausnehmung 6a aufweist, als dies beim Bekannten nach US-B2 bekannt der Fall ist. Dadurch ergibt sich - insbesondere im Vergleich zu herkömmlichen geschlitzten Hämmern - eine geringere Vibrationsgefahr, wenn mit der oberen Grundfläche 5a geschlagen wird.

Aus Fig. 1 wird deutlich, dass der Einführkanal 7a und der Verriegelungsraum 9a nicht parallel zueinander angeordnet sind, sondern winkelig. Dieser Winkel beträgt ca. 20-160°, insbesondere ca. 30 - 150°.

Bevorzugt ist dabei erfindungsgemäss, dass der Winkel jedenfalls so gross gewählt ist, dass die Führungsstange 17 bei normalem Gleit-Schlagbetrieb nicht über den Axialkanal 8a wieder herausrutscht.

Im Ausführungsbeispiel der Fig. 1 sind Einführkanal 7a und Axialkanal 8a zu dem Verriegelungsraum 9a in einem anderen Winkel angeordnet. Ebenso kann jedoch der Einführkanal 7a zum Axialkanal 8a und dem Verriegelungsraum 9a in einem anderen Winkel angeordnet sein. Vorteilhaft ist lediglich, dass der Einführkanal 7a und der Verriegelungsraum 9a in einem Winkel zueinander angelegt sind und durch einen dritten Bereich, als Axialkanal 8a bezeichnet, miteinander verbunden sind.

Aus der Zusammenschau mit der Bewegungsdarstellung in Fig. 2 wird dies deutlich. Fig. 2A zeigt die Bewegung, die beim Einführen der Führungsstange 17 vollzogen wird, wenn der chirurgische Hammer bzw. die Ausnehmung 6a entsprechend der Fig. 1 gestaltet ist. Sämtliche Bezeichnungen der Bewegungsrichtung beziehen sich in der Beschreibung der Fig. 2 auf die Längsachse 11 des Hammerkopfes 2a aus Fig. 1.

Beim Einführen der Führungsstange 17 in die Ausnehmung 6a wird folglich zunächst eine Radialbewegung 14 vollzogen. Anschliessend wird die Führungsstange 17 parallel zur Längsachse 11 des Hammerkopfes 2a geführt; es wird eine Axialbewegung 15 vollzogen. Im Anschluss daran, wird nach Erreichen der Ebene des Verriegelungsraums 9a eine Rotationsbewegung 16 vollzogen.

In der anderen Ausgestaltung, dargestellt in Fig. 2B, wird zunächst eine Rotationsbewegung 16 vollzogen. Das heisst, die Führungsstange 17 wird in den Einführkanal eingeführt und dann sofort gedreht. Anschliessend wird wiederum eine Axialbewegung 15 ausgeführt, an die sich eine Radialbewegung 14 anschliesst. Als dritte Alternative wäre auch möglich, den Axialkanal so zu gestalten, dass durch die Führung im Axialkanal die Rotationsbewegung vollbracht wird, so dass die Bewegungsrichtungen im Einführkanal und im Verriegelungsraum stets radial sind, während die Bewegungen im Axialkanal sowohl axial als auch rotatorisch erfolgt. Die Rotationsbewegung in der Ausführung der Fig. 1 erfolgt dabei stets um die Längsachse des Hammerkopfes.

Es wird deutlich, dass auch andere Ausgestaltungen des Hammerkopfes bzw. der Ausnehmung im Hammerkopf möglich sind, solange mehrere gesonderte Bereiche vorgesehen sind, durch die nach dem Einführen der Führungsstange 17 eine Verriegelung ermöglicht wird, die eine gute Führung gewährleistet.

Aus Fig. 1 wird weiter deutlich, dass - für den Hammerbetrieb ohne Führungsstange - als Schlagflächen die Grundflächen 5a erhalten bleiben. Die Ausnehmung 6a ist im Mantelbereich des zylindrischen Hammerkopfes 2a vorgesehen. Die Grundflächen 5a bleiben unversehrt. Die Grundfläche 5a ist folglich in sich geschlossen.

Eine alternative Ausführung, die nicht Teil der Erfindung ist, sieht jedoch vor, dass die Ausnehmung in der Grundfläche 5a und in der Mantelfläche 4a ausgebildet ist. In diesem Fall befindet sich der Axialkanal 8 annähernd senkrecht zu der Längsachse 11 des Hammerkopfes 2. Anders ausgedrückt ist der Axialkanal 8 radial angeordnet. Im Ausführungsbeispiel der Fig. 1 ist dagegen der Axialkanal 8a annähernd koaxial zur Längsachse 11 des Hammerkopfes 2a angeordnet.

Das Vorsehen eines Hammerstieles 3 ist lediglich eine bevorzugte Ausführungsform. In Verbindung mit einer Führungsstange ist es grundsätzlich auch möglich, wie im Stand der Technik bekannt, lediglich einen Hammerkopf 2 auszubilden, ohne einen Hammerstiel 3. Um jedoch den chirurgischen Hammer 1 mit all seinen Vorteilen nutzen zu können, ist die Ausbildung eines Hammerstieles 3 bevorzugt. Dieser verfügt bevorzugt über einen anatomisch geformten Griff mit z.B. zwei etwa parallelen Griffflächen, die je in einer Normalebene auf die Grundflächen 5b liegen.

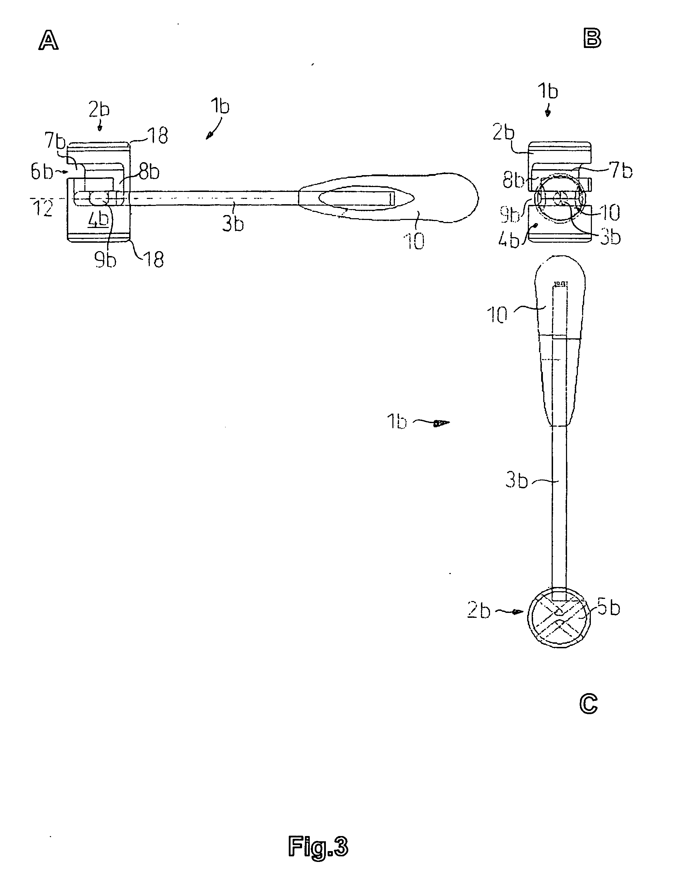

In Fig. 3 sind unterschiedliche Ansichten eines bevorzugten Ausführungsbeispiels des chirurgischen Hammers 1 b dargestellt. Die Fig. 3A zeigt den chirurgischen Hammer 1b in Seitenansicht, Fig. 3B in Vorderansicht und Fig. 3C in Aufsicht. Der Hammer 1b weist einen Hammerkopf 2b und einen Stiel 3b auf. Hammerkopf 2 und Stiel 3 sind bevorzugt miteinander verschweisst. Am Stiel 3b ist ein Griff 10 vorgesehen. Der Griff 10 ist vorzugsweise durch Stiften mit dem Stiel 3 verbunden. Der Griff 10 weist bevorzugt wenigstens eine abgeflachte Seite auf, wodurch der Griff verbessert wird und wodurch erreicht wird, dass der erfindungsgemässe Hammer 1 stets in der richtigen Stellung in der Hand liegt.

Der Griff 10 ist vorzugsweise aus einem vergleichsweise warmen und griffigen Material gebildet, das jedoch gut sterilisierbar ist, beispielsweise aus einem Kunststoffmaterial.

Der Hammerkopf 2b und der Stiel 3b bestehen vorzugsweise aus einem metallischen Werkstoff, der für medizinische Zwecke geeignet ist, beispielsweise 5CrNiCuNb16-4.

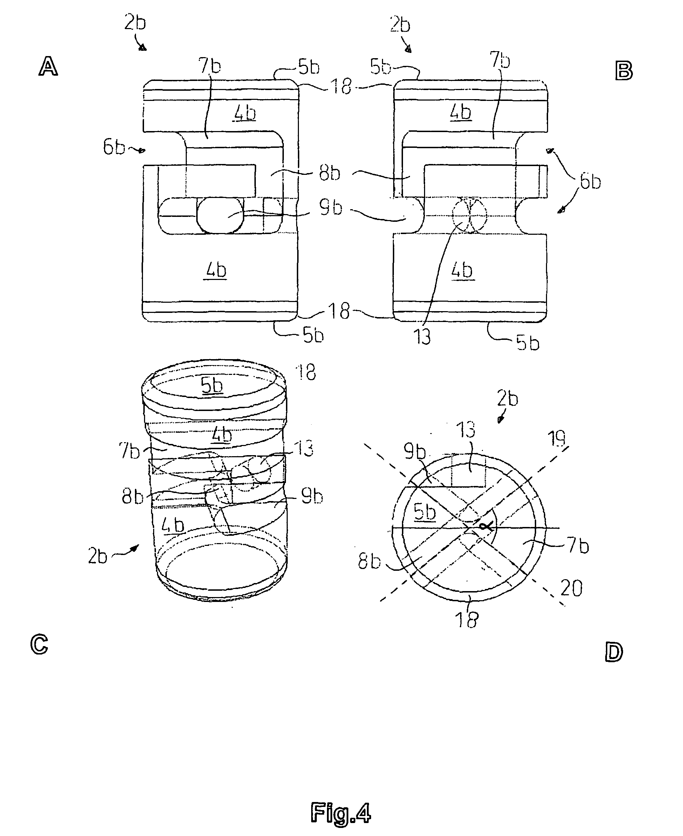

Aus einer Zusammenschau der Fig. 3 und 4 ergibt sich ferner die Gestaltung des beispielhaften Hammerkopfes 2b. In Fig. 4 sind unterschiedliche Ansichten des Hammerkopfes 2b dargestellt. Fig. 4A zeigt den Hammerkopf in Seitenansicht, Fig. 4B in Vorderansicht, Fig. 4C in einer perspektivischen Gesamtdarstellung und Fig. 4D in Aufsicht.

Die Darstellungen sind stets so gewählt, dass auch im Inneren des Hammerkopfes 2b liegende Linien und Elemente dargestellt sind.

Der Hammerkopf 2b weist eine im Wesentlichen zylindrische Gestalt auf. Vorzugsweise sind zwischen Mantelfläche 4b und den Grundflächen 5b gerundete Kanten 18 vorgesehen, wodurch sich ein Unterschied zum Aufbau nach Fig.1 ergibt.

Deutlich wird die Anordnung der Ausnehmung 6b. Die Ausnehmung 6b ist wiederum durch einen Einführkanal 7b, einen Axialkanal 8b und einen Verriegelungsraum 9b gebildet. Insbesondere aus den Aufsichten der Fig. 3C und 4D wird deutlich, dass der Einführkanal 7b und der Verriegelungsraum 9b winkelig zueinander angeordnet sind. In Fig. 4D ist zum einen die Längsachse der Führungsstange im Einführkanal 7 eingetragen, bezeichnet mit dem Bezugszeichen 19.

Zum anderen ist in Fig. 4D die Längsachse der Führungsstange im Verriegelungsraum 9b eingetragen, bezeichnet mit dem Bezugszeichen 20.

Im Ausführungsbeispiel der Fig. 3 und 4 beträgt der Winkel α ungefähr 80°. Dies stellt jedoch lediglich eine bevorzugte Ausführung dar.

Aus den Fig. 3 und 4 ergibt sich ferner die Anordnung des Hammerstieles 3b am Hammerkopf 2b. Es wird deutlich, dass der Hammerstiel 3b aussermittig angeordnet ist. Der Hammerstiel 3b ist insbesondere so angeordnet, dass der Schwerpunkt des Hammerstielkopfes durch die Verlängerung der Hammerstielachse 12 hindurchgeht. Bedingt durch die Ausnehmung 6b wird ein Bereich des Hammerkopfes 2b nämlich leichter als der andere Bereich.

In der Darstellung der Fig. 4 ist der obere Bereich des Hammerkopfes 2b mit der Ausnehmung 6b versehen, und somit leichter als der untere Bereich, der überwiegend massiv gestaltet ist. Folglich ist der Ansatz 13 des Hammerstieles 3b in Richtung zum, in der Abbildung dargestellten, unteren Bereich des Hammerkopfes 2b hin verschoben.

Deutlich wird ferner, dass sich Verriegelungsraum 9b und Ansatz 13 des Hammerstieles 3 annähernd in derselben Ebene befinden. Auch dies ist als vorteilhaft anzusehen, da die Führung über die Führungsstange 17 erleichtert wird.

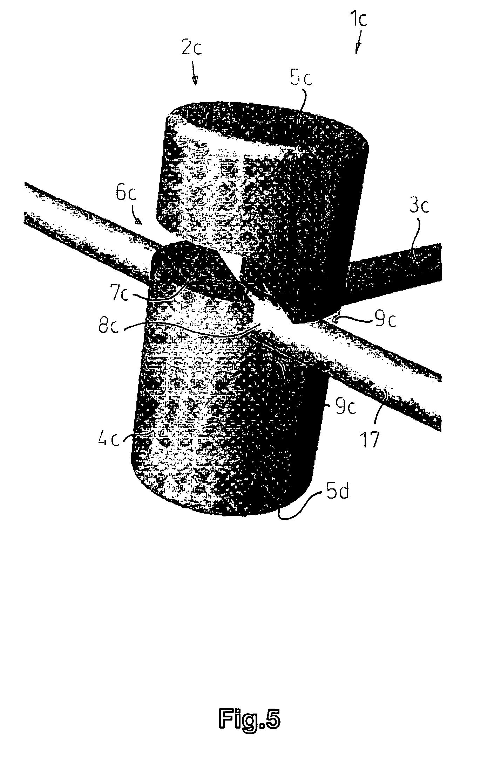

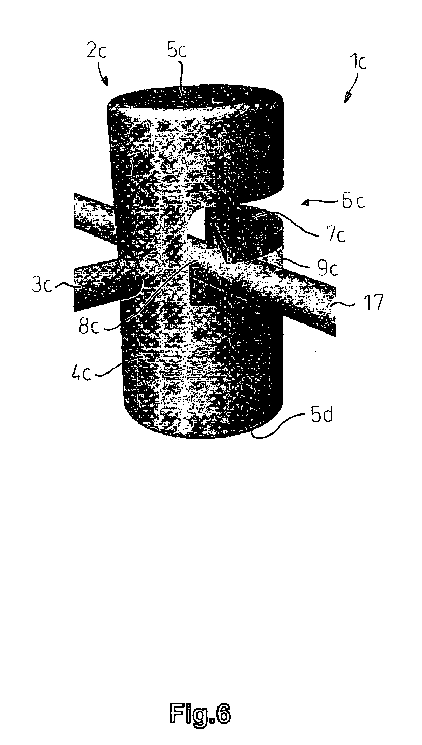

In den Fig. 5 (Vorderansicht) und 6 (Rückansicht) ist der erfindungsgemässe Hammer 1 c perspektivisch dargestellt. Er weist ebenfalls einen Hammerkopf 2c und einen Hammerstiel 3c, wiederum nur teilweise gezeigt, auf. Die Führungsstange 17 ist in den Fig. 5 und 6 in der Position dargestellt, in der sie verriegelt ist und in welcher der Hammerkopf 2c über sie bewegt wird, um die Extraktion oder die Insertion eines Implantates über eine Führungsstange 17 zu erzielen.

Deutlich wird die Anordnung von Einführkanal 7c, Axialkanal 8c und Verriegelungsraum 9c. Im Hammerkopf 2c sind somit drei Bereiche ausgebildet: ein mittlerer Bereich, in dem sich der Einführkanal 7c, der Axialkanal 8c und der Verriegelungsraum 9c befindet, der umgeben ist von je einem Bereich, der durchbrechungsfrei und damit massiv gestaltet ist.

Die Grundflächen 5c und 5d stellen somit ausgezeichnete, massive und von Durchbrechungen freie Schlagflächen dar, wobei insbesondere die Grundfläche 5d besonders gut als solche geeignet ist. Gut zu sehen ist auch, dass die Grundflächen 5c und 5d unversehrt sind, da die Ausnehmung 6c in der Mantelfläche 4c ausgebildet ist.

Nachfolgend soll kurz die Funktionsweise des erfindungsgemässen Hammers beschrieben werden.

Bei Verwenden des Hammers 1 in axialer Richtung, wobei wiederum die Längsachse des Hammerkopfes 2 als Massstab herangezogen wird, ist der Hammer 1 in üblicher Art und Weise zu benutzen, indem die Grundflächen 5 als Schlagflächen verwendet werden. Nach dem Drehen um 90° um die Stielrichtung kann der Hammerkopf 2 auf eine Führungsstange 17 aufgefädelt werden. Durch das Kippen des Hammers 1 kann die Ausnehmung 6 über den Einführkanal 7 über die Führungsstange 17 geführt werden. Der Hammerkopf wird nun axial im Axialkanal 8 verschoben, solange bis der Verriegelungsraum 9 erreicht ist. In diesem Bereich ist nun eine Rotationsbewegung des Hammerkopfes 2 um die Führungsstange 17 herum möglich. Es schliesst sich folglich eine Rotation in einem Winkel zwischen +60°/-60°, bevorzugt zwischen +40°/-40° an.

Alternativ könnte die Fräsung im Hammerkopf, durch die der Verriegelungsraum 9 gebildet wird, auch asymmetrisch ausgebildet sein, beispielsweise in einem Winkel von +40°/-50°. Durch die leichte Asymmetrie wird die Verriegelung der Führungsstange 17 im Verriegelungsraum 9 gegebenenfalls verbessert.

Der Verriegelungsraum und der Einführkanal sollten ca. 10 mm voneinander beabstandet sein, wenn sie im Wesentlichen parallel laufen, damit bei dem gewählten Hammermaterial ausreichend Masse zwischen den beiden Räumen gegeben ist, und der Hammer ausreichend stabil ist.

Der Ansatz 13 des Hammerstiels 3 und der Einführkanal 7 können ungefähr im gleichen Abstand vom Rand des Hammerkopfes 2 angebracht sein. Der Axialkanal 8 bildet die Verbindung zwischen dem Einführkanal 7 und dem Verriegelungsraum 9 und sollte den gleichen gewählten Durchmesser, wie zum Beispiel 10,5 mm haben.

Die Kanten des Axialkanals 8 und des Verriegelungsraums 9 sollten abgerundet sein; eine brauchbare Abrundung könnte ca. 5 mm betragen.

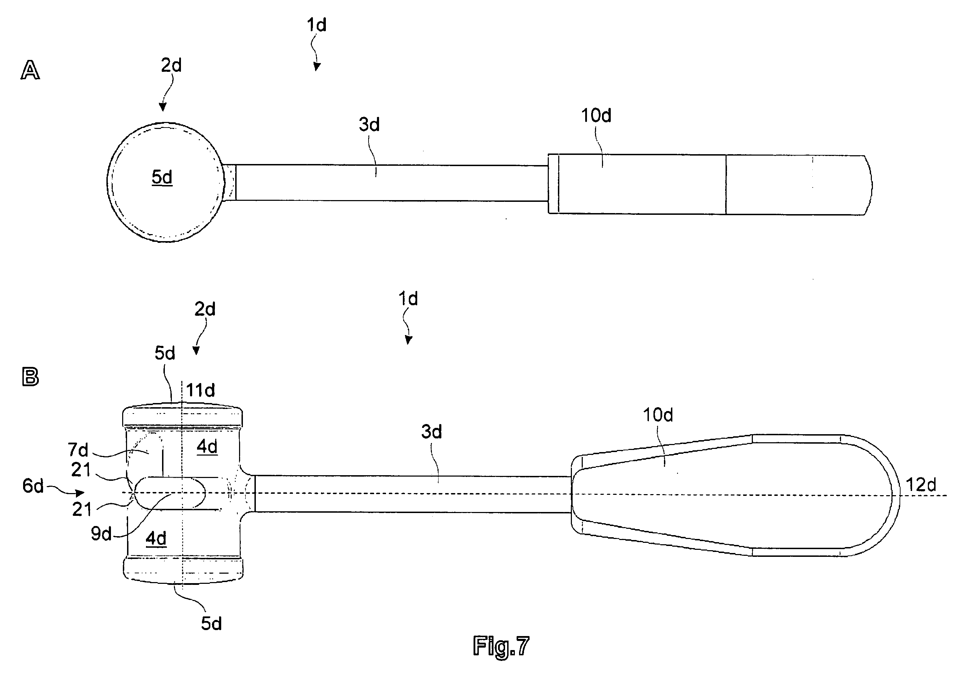

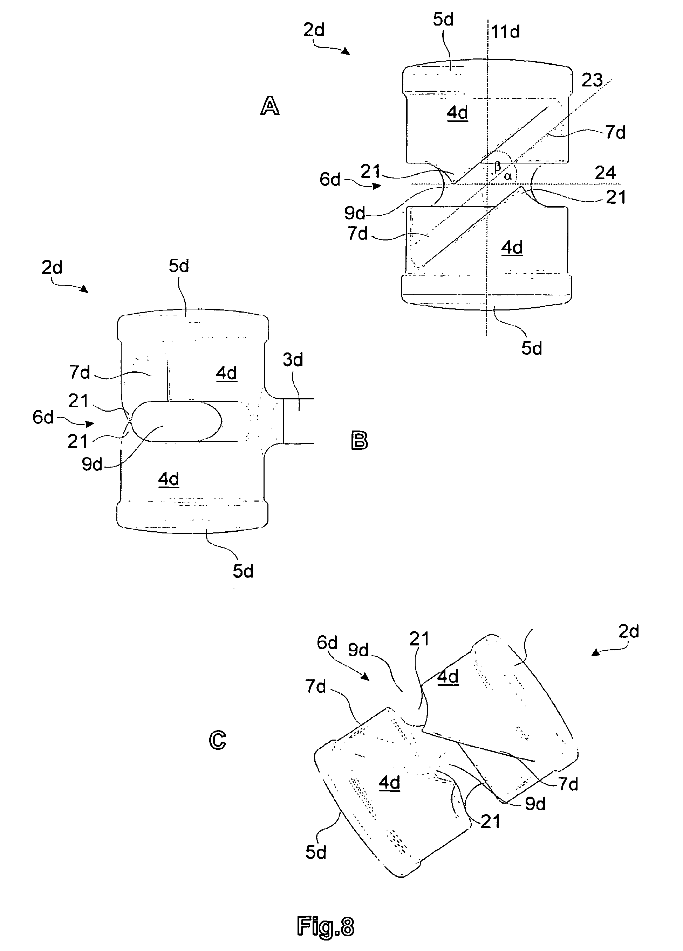

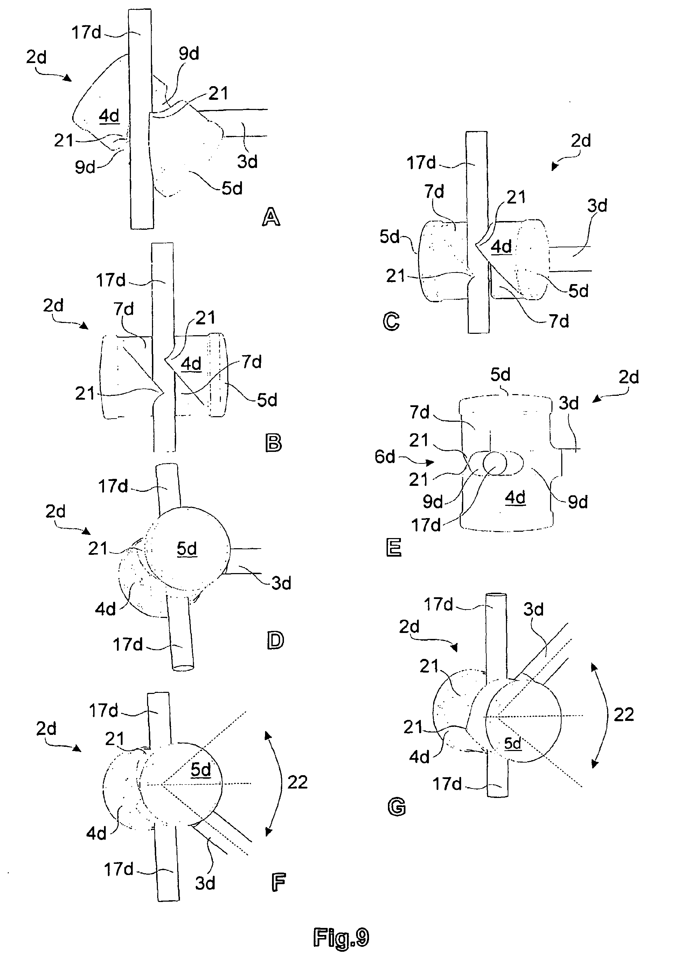

In den Fig. 7-9 ist ein weiteres Ausführungsbeispiel eines erfindungsgemässen chirurgischen Hammers 1d dargestellt. Die Fig. 7 zeigt dabei den Hammer 1d in Aufsicht (A) und in Seitenansicht (B). In Fig. 8 ist der Hammerkopf 2d in verschiedenen Ansichten dargestellt. Die Fig. 8A und 8C sind Vorderansichten und Fig. 8B ist eine Seitenansicht. Fig. 9 zeigt die Funktionsweise des chirurgischen Hammers im Detail. Hierfür sind verschiedene Stadien des Einführens der Führungsstange 17d in verschiedenen Ansichten dargestellt. In den Fig. 9A-C ist der Hammerkopf 2d jeweils in perspektivischer Vorderansicht gezeigt. Die Fig. 9D, F und G zeigen jeweils Aufsichten und Fig. 9G zeigt eine perspektivische Seitenansicht.

Der chirurgische Hammer 1d weist einen Hammerkopf 2d und einen Stiel 3d auf, welcher einen Griff 10d hat. Der Griff 10d ist bevorzugt abgeflacht gestaltet, wie aus einem Vergleich der Darstellungen der Fig. 7A und 7B ersichtlich ist. Die Längsachse 12d des Hammers 1d ist durch eine gestrichelte Linie in Fig. 7B eingetragen worden.

Aus einem Vergleich der Darstellungen der Fig. 7A und 7B wird weiter ersichtlich, dass die Grundfläche 5d in sich geschlossen ist. Die Ausnehmung 6d erstreckt sich nicht bis in die Grundfläche 5d hinein sondern ist auf den Bereich der Mantelfläche 4d beschränkt. Dadurch ergibt sich vorteilhaft, dass beide Schlagflächen des Hammers 1d voll erhalten bleiben. Der erfindungsgemässe Hammer kann folglich auch als "normaler" Hammer, d. h. zum Schlagen, eingesetzt werden; anders als aus dem Stand der Technik bekannte Hämmer. Der Chirurg braucht folglich nur einen Hammer mit dem er mit aufgefädelter Führungsstange die Insertion/Extraktion durchführen kann und mit dem er auch Schlagen kann, wie mit jedem gewöhnlichen Hammer.

Das Ausführungsbeispiel der Fig. 7-9 zeigt eine weitere Ausführung einer Ausnehmung 6d. Diese weist einen Einführkanal 7d und einen Verriegelungsraum 9d auf. Der Verriegelungsraum 9d ist radial ausgerichtet. Er verläuft annähernd horizontal oder, anders ausgedrückt, annähernd senkrecht zur Längsachse 11d des Hammerkopfs.

Der Einführkanal 7d und der Verriegelungsraum 9d sind zueinander in einem bestimmten Winkel α angeordnet, wie aus der Fig. 8A deutlich wird. Die Längsachse 24 des Verriegelungsraumes 9d und die Längsachse 23 des Einführkanals 7d schliessen den Winkel α ein, der ca. 20-70°, bevorzugt 40-50° beträgt. Im Ausführungsbeispiel der Fig. 6-8 beträgt er ca. 50°. Weiterhin schliessen die Längsachse 23 des Einführkanals 7d und die Längsachse 11d des Hammerkopfes 2d den Winkel β ein. Dieser liegt in einem Bereich von ca. 20-70°, bevorzugt 40-50°. Im Ausführungsbeispiel der Fig. 7-9 beträgt er ca. 40°. Der Einführkanal 7d ist folglich auf dem Hammerkopf 2d relativ zu dessen Längsachse 11 d schräg angeordnet, wie aus den Fig. 7-9 deutlich ersichtlich ist. Der Einführkanal 7d ist so ausgeformt, dass er das Einführen der Führungsstange 17d erlaubt.

Der Verriegelungsraum 9d ist langlochförmig gestaltet. In diesem Schlitz läuft die Führungsstange 17d. Der Verriegelungsraum 9d ist überdies schräg ausgeformt, so dass es möglich ist, die Führungsstange 17d um das Zentrum des Hammerkopfes zu drehen, wie aus den Fig. 9F und 9G ersichtlich ist. Zur Aussenseite hin ist der Verriegelungsraum 9d durch mindestens einen, bevorzugt zwei Vorsprünge 21 begrenzt. Diese Vorsprünge 21 sind zueinander versetzt angeordnet. Genauer ist je ein Vorsprung 21 an je einer Seite des Einführkanals 7d angeordnet, wie beispielsweise aus Fig. 8A deutlich wird.

Aus der Figurenfolge der Fig. 9 wird die Funktionsweise des Hammerkopfes 2d im Detail deutlich. Die Führungsstange 17d ist dabei mit Ausnahme der Fig. 9E stets vertikal ausgerichtet. Zum Einführen wird der Hammerkopf 2d zunächst schräg auf die Führungsstange 17d aufgesetzt, so dass diese in den Einführkanal 7d eingeführt werden kann (Fig. 9A). Dann wird der Hammerkopf 2d in die horizontale Position gedreht (Fig. 9B, 9C). Nun wird der Hammerkopf 2d solange weitergeschoben bis die Führungsstange 17d in einer zentralen Position zu liegen kommt (Fig. 9D, 9E). In diesem Zustand ist der Hammerkopf 2d auf die Führungsstange 17d aufgefädelt. Der Chirurg kann nun durch eine Auf- und Ab-Bewegung die Führungsstange betätigen, d.h. das Implantat inserieren oder extrahieren, je nach Bedarf.

Aus den Fig. 9B und 9C wird deutlich, dass die Führungsstange 17d in dem Verriegelungsraum 9d durch die Vorsprünge 21 verriegelt ist. Die Vorsprünge 21 begrenzen den Verriegelungsraum 9d nach aussen hin. Durch die erfindungsgemässe Gestaltung der Ausnehmung 6d stellen die Vorsprünge 21 jedoch beim Einführen der Führungsstange 17d kein Hindernis dar.

Aus den Fig. 9F und 9G wird der Bewegungsspielraum deutlich, den der Chirurg bei der Benutzung des Hammers 1d bei der Insertion bzw. Extraktion von Implantaten, z. B. intermedullären Nägeln, hat. Der Hammerkopf kann über der Führungsstange 17d in einem grossen Winkel von ca. 80° um das Zentrum des Hammerkopfes bewegt werden, wie angedeutet durch den Pfeil 22. Dadurch kann der Chirurg eine bogenförmige Bewegung beim Inserieren bzw. Extrahieren ausüben, was der natürlichen Schlagbewegung sehr nahe kommt.

Der erfindungsgemässe Hammerkopf 2 lässt dem Chirurgen also einerseits einen grossen Spielraum bei dem Betätigen der Vorrichtung zum Inserieren und/oder Extrahieren, andererseits gewährt er eine exakte Führung und verhindert das Abrutschen des Hammerkopfes 2 von der Vorrichtung zum Inserieren und/oder Extrahieren. Schliesslich ist der Hammer 1 auch in der Art eines gewöhnlichen Hammers zu verwenden, so dass er universell einsetzbar ist.

In einer vorteilhaften Ausgestaltung ist der Hammerkopf 2 an seinen Rändern mit einem Radius von ca. 3 mm abgerundet worden. Die Bohrung für den Stiel im Hammerkopf hat ein Mass von ca. 10 mm und reicht ca. 10 mm in den Hammerkopf hinein. Der Mantel des Hammerkopfes hat eine gesamte Höhe von beispielsweise ca. 73,5 mm, wobei die Bohrung für den Stiel ca. 32 mm bis ca. 33 mm von einem Rand des Hammerkopfes entfernt in dem Hammerkopf angebracht ist.

Wenn die Vorrichtung für die Insertion oder Extraktion von Implantaten rund ist und einen Durchmesser von ca. 10 mm hat, so sollte die Ausnehmung 6 in einem Bereich von ca. 10,5 mm liegen, um eine sichere Führung zu bieten und gleichzeitig einen sicheren Halt bei genügend Spiel zu bieten. Alle Ausnehmungen 6 sind bevorzugt gerundet.

Damit der Griff 10 angenehm in der Hand liegt, sollte er ca. 120 mm bis ca. 130 mm, z. B. ca. 129 mm lang sein, während der gesamte Stiel mit Griff ungefähr die doppelte Länge, also 260 mm, lang sein sollte.

### Bezugszeichenliste

- 1 -: chirurgischer Hammer
- 2 -: Hammerkopf
- 3 -: Hammerstiel
- 4 -: Mantelfläche
- 5 -: Grundfläche
- 6 -: Ausnehmung
- 7 -: Einführkanal
- 8 -: Axialkanal
- 9 -: Verriegelungsraum
- 10-: Griff
- 11 -: Längsachse Hammerkopf
- 12 -: Längsachse Hammerstiel
- 13 -: Ansatz des Hammerstiels
- 14 -: Radialbewegung
- 15 -: Axialbewegung
- 16 -: Rotationsbewegung
- 17 -: Führungsstange
- 18 -: abgerundete Kante
- 19 -: Längsachse der 17 im 7
- 20 -: Längsachse der 17 im 9
- 21 -: Vorsprung
- 22 -: Pfeil
- 23 -: Längsachse von 7
- 24 -: Längsachse von 9

## Patentansprüche

1. Chirurgischer Hammer (1), aufweisend einen Hammerkopf (2), wobei der Hammerkopf eine im Wesentlichen zylindrische Gestalt aufweist und mit zumindest einer Ausnehmung (6) versehen ist, in der eine Vorrichtung (17) zum Inserieren und/oder Extrahieren von Implantaten, insbesondere von intermedullären Nägeln und Kirschner-Drähten, aufnehmbar ist, wobei die Ausnehmung (6) durch mindestens zwei gesonderte Bereiche gebildet ist, nämlich durch Einführkanal (7) und Verriegelungsraum (9), wobei die Vorrichtung (17) zum Inserieren und/oder Extrahieren im Verriegelungsraum verriegelbar ist, **dadurch gekennzeichnet, dass** die Grundfläche (5) des Hammerkopfes in sich geschlossen ist.

2. Chirurgischer Hammer nach Anspruch 1, **dadurch gekennzeichnet, dass** sich der Verriegelungsraum (9) im Wesentlichen in radialer Richtung erstreckt.

3. Chirurgischer Hammer nach Anspruch 1 oder 2, **dadurch gekennzeichnet, dass** Einführkanal (7) und Verriegelungsraum (9) in einem Winkel (α) von annähernd ca. 10-160°, insbesondere von ca. 20-150° zueinander angeordnet sind.

4. Chirurgischer Hammer nach einem der vorhergehenden Ansprüche, **dadurch gekennzeichnet, dass** die Ausnehmung (6) zusätzlich einen Axialkanal (8) aufweist **und dass** der Einführkanal (7) und der Axialkanal (8) zu dem Verriegelungsraum (9) in einem Winkel (α) von annähernd ca. 20-160°, insbesondere von ca. 30-150° angeordnet sind.

5. Chirurgischer Hammer nach einem der vorhergehenden Ansprüche, **dadurch gekennzeichnet, dass** der Einführkanal (7) zu dem Axialkanal (8) und dem Verriegelungsraum (9) in einem Winkel (α) von annähernd ca. 20-160°, insbesondere ca. 30-150° angeordnet ist.

6. Chirurgischer Hammer nach einem der Ansprüche 3 bis 5, **dadurch gekennzeichnet, dass** der Winkel (α) ca. 80° beträgt.

7. Chirurgischer Hammer nach einem der vorhergehenden Ansprüche, **dadurch gekennzeichnet, dass** die Ausnehmung (6) so gestaltet ist, dass der Hammer durch eine Radial(14)-, Axial(15)- und Rotations(16)bewegung in Bezug auf die Längsachse (11) des Hammerkopfes (2) einführbar ist.

8. Chirurgischer Hammer nach einem der vorhergehenden Ansprüche, **dadurch gekennzeichnet, dass** der Axialkanal (8) mit seiner mittleren Axialebene wenigstens annähernd in der Längsachse (11) des Hammerkopfes (2) angeordnet ist.

9. Chirurgischer Hammer nach einem der Ansprüche 4 bis 7, **dadurch gekennzeichnet, dass** der Axialkanal (8) wenigstens annähernd senkrecht zu der Längsachse (11) des Hammerkopfes (2) angeordnet ist.

10. Chirurgischer Hammer nach einem der Ansprüche 1 bis 3, **dadurch gekennzeichnet, dass** der Einführkanal (7) in einem Winkel (β) von annähernd ca. 20-70°, insbesondere von ca. 40-50° zu der Längsachse (11) des Hammerkopfes (2) angeordnet ist.

11. Chirurgischer Hammer nach Anspruch 3 oder 10, **dadurch gekennzeichnet, dass** Einführkanal (7) und Verriegelungsraum (9) in einem Winkel (α) von annähernd ca. 20-70°, insbesondere von ca. 30-40° zueinander angeordnet sind.

12. Chirurgischer Hammer nach einem der Ansprüche 1, 2, 3, 10 oder 11, **dadurch gekennzeichnet, dass** der Verriegelunsraum (9) durch mindestens einen, bevorzugt mehrere Vorsprünge (21) begrenzt wird, wobei diese Vorsprünge insbesondere zueinander versetzt angeordnet sind.

13. Chirurgischer Hammer nach einem der Ansprüche 1, 2, 3, 10, 11 oder 12, **dadurch gekennzeichnet, dass** der Verriegelungsraum (9) langlochförmig ist.

14. Chirurgischer Hammer nach einem der vorhergehenden Ansprüche, **dadurch gekennzeichnet, dass** die Schlagrichtung des Hammers (1) bei eingeführter Vorrichtung (17) zum Inserieren und/oder Extrahieren im Wesentlichen senkrecht zur Längsachse (11) des Hammerkopfes verläuft, wobei sich der Hammerkopf (2) während der Schlagbewegung vorzugsweise um seine Längsachse (11) drehen kann .

15. Chirurgischer Hammer nach einem der vorhergehenden Ansprüche, **dadurch gekennzeichnet, dass** am Hammerkopf (2) ein Hammerstiel (3) befestigt ist.

16. Chirurgischer Hammer nach einem der vorhergehenden Ansprüche, **dadurch gekennzeichnet, dass** die Ausnehmung (6) so angelegt ist, dass sich der Schwerpunkt des Hammers (1) annähernd in der Verlängerung der Längsachse (12) des Hammerstiels (3) befindet.

17. Chirurgischer Hammer nach einem der Ansprüche 15 oder 16, **dadurch gekennzeichnet, dass** der Hammerstiel (3) am Hammerkopf (2) aussermittig angeordnet ist.

18. Chirurgischer Hammer nach einem der vorangegangenen Ansprüche, **dadurch gekennzeichnet, dass** im Verriegelungsraum (9) des Hammerkopfes (2) zusätzliche Verriegelungselemente, wie z. B. Kugeldruckstifte o. dgl., zur zusätzlichen Arretierung an einer Vorrichtung (17) zum Inserieren und/oder Extrahieren vorgesehen sind.

19. Chirurgischer Hammer nach einem der Ansprüche 15 bis 18, **dadurch gekennzeichnet, dass** der Hammerstiel (3) einen Griff (10) aufweist, insbesondere einen Griff, der an wenigstens einer Seite abgeflacht ist.

## Claims

1. Surgical mallet (1), having a mallet head (2), the mallet head having a substantially cylindrical shape and being provided with at least one recess (6) in which a device (17) for insertion and/or extraction of implants, in particular of intramedullary nails and Kirschner wires can be held, whereas the recess (6) is formed by at least two separate regions, namely by insertion channel (7) and locking chamber (9), the device (17) for insertion and/or extraction being capable of being locked in the locking chamber, **characterized in that** the base surface (5) of the mallet head is cohesive.

2. Surgical mallet according to Claim 1, **characterized in that** the locking chamber (9) extends substantially in radial direction.

3. Surgical mallet according to Claim 1 or 2, **characterized in that** insertion channel (7) and locking chamber (9) are arranged at an angle (α) of approximately 10-160°, in particular of approximately 20-150° to one another.

4. Surgical mallet according to any of the preceding Claims, **characterized in that** the recess (6) additionally has an axial channel (8), and **in that** the insertion channel (7) and the axial channel (8) are arranged at an angle (α) of approximately 20-160°, in particular of approximately 30-150° to the locking chamber (9).

5. Surgical mallet according to any of the preceding Claims, **characterized in that** the insertion channel (7) is arranged at an angle (α) of approximately 20-160°, in particular approximately 30-150° to the axial channel (8) and the locking chamber (9).

6. Surgical mallet according to any of Claims 3 to 5, **characterized in that** the angle (α) is approximately 80°.

7. Surgical mallet according to any of the preceding Claims, **characterized in that** the recess (6) is formed in such a manner that the mallet can be inserted by a radial (14), axial (15) and rotational (16) movement relative to the longitudinal axis (11) of the mallet head (2).

8. Surgical mallet according to any of the preceding Claims **characterized in that** the axial channel (8) is arranged with its central axial plane at least approximately in the longitudinal axis (11) of the mallet head (2).

9. Surgical mallet according to any of Claims 4 to 7, **characterized in that** the axial channel (8) is arranged at least approximately perpendicularly to the longitudinal axis (11) of the mallet head (2).

10. Surgical mallet according to any of Claims 1 to 3, **characterized in that** the insertion channel (7) is arranged at an angle (β) of approximately 20-70°, in particular of approximately 40-50° to the longitudinal axis (11) of the mallet head (2).

11. Surgical mallet according to Claim 3 or 10, **characterized in that** insertion channel (7) and locking chamber (9) are arranged at an angle (α) of approximately 20-70°, in particular of approximately 30-40°, to one another.

12. Surgical mallet according to any of Claims 1, 2, 3, 10 or 11, **characterized in that** the locking chamber (9) is bounded by at least one projection (21), preferably a plurality of projections (21), these projections being arranged in particular staggered relative to one another.

13. Surgical mallet according to any of Claims 1, 2, 3, 10, 11 or 12, **characterized in that** the locking chamber (9) is in the form of an elongated hole.

14. Surgical mallet according to any of the preceding Claims, **characterized in that** the impact direction of the mallet (1) when the device (17) for insertion and/or extraction has been introduced is substantially perpendicular to the longitudinal axis (11) of the mallet head, it being possible for the mallet head (2) to rotate during the striking movement, preferably about its longitudinal axis (11).

15. Surgical mallet according to any of the preceding Claims, **characterized in that** a mallet shaft (3) is fixed to the mallet head (2).

16. Surgical mallet according to any of the preceding Claims, **characterized in that** the recess (6) is positioned in such a way that the centre of gravity of the mallet (1) is present approximately in the extrapolation of the longitudinal axis (12) of the mallet shaft (3).

17. Surgical mallet according to any of Claims 15 or 16, **characterized in that** the mallet shaft (3) is arranged out of centre on the mallet head (2).

18. Surgical mallet according to any of the preceding Claims, **characterized in that** additional locking elements, such as, for example, spherical pressure pins or the like, for additional locking on a device (17) for insertion and/or extraction are provided in the locking chamber (9) of the mallet head (2).

19. Surgical mallet according to any of Claims 15 to 18, **characterized in that** the mallet shaft (3) has a handle (10), in particular a handle which is flattened on at least one side.

## Revendications

1. Marteau chirurgical (1), comportant une tête de marteau (2), la tête de marteau étant essentiellement de forme cylindrique et dotée d'au moins un évidement (6) dans lequel un dispositif (17) pour l'insertion et/ou l'extraction d'implants, en particulier d'ongles intramédullaires et de fils Kirschner, peut être logé, l'évidement (6) étant formé par au moins deux zones séparées, à savoir par le canal d'introduction (7) et par l'espace de verrouillage (9), le dispositif (17) pour l'insertion et/ou l'extraction étant verrouillable dans l'espace de verrouillage, **caractérisé en ce que** la surface de base (5) de la tête du marteau est elle-même fermée.

2. Marteau chirurgical selon la revendication 1, **caractérisé en ce que** l'espace de verrouillage (9) s'étend essentiellement dans le sens radial.

3. Marteau chirurgical selon la revendication 1 ou 2, **caractérisé en ce que** le canal d'introduction (7) et l'espace de verrouillage (9) sont disposés dans un angle (α) approximativement d'environ 10 à 160°, en particulier d'environ 20 à 150°, l'un par rapport à l'autre.

4. Marteau chirurgical selon une des revendications précédentes, **caractérisé en ce que** l'évidement (6) comporte en outre un canal axial (8) et **en ce que** le canal d'introduction (7) et le canal axial (8) sont disposés dans un angle (α) approximativement d'environ 20 à 160°, en particulier d'environ 30 à 150°, par rapport à l'espace de verrouillage (9).

5. Marteau chirurgical selon une des revendications précédentes, **caractérisé en ce que** le canal d'introduction (7) est disposé dans un angle (α) approximativement d'environ 20 à 160°, en particulier d'environ 30 à 150°, par rapport au canal axial (8) et à l'espace de verrouillage (9).

6. Marteau chirurgical selon une des revendications 3 à 5, **caractérisé en ce que** l'angle (α) est d'environ 80°.

7. Marteau chirurgical selon une des revendications précédentes, **caractérisé en ce que** l'évidement (6) est configuré de telle manière que le marteau peut être introduit par un mouvement radial (14), axial (15) et de rotation (16) par rapport à l'axe longitudinal (11) de la tête du marteau (2).

8. Marteau chirurgical selon une des revendications précédentes, **caractérisé en ce que** le canal axial (8) avec son plan axial médian est disposé au moins approximativement dans l'axe longitudinal (11) de la tête du marteau (2).

9. Marteau chirurgical selon une des revendications 4 à 7, **caractérisé en ce que** le canal axial (8) est disposé au moins approximativement perpendiculairement à l'axe longitudinal (11) de la tête du marteau (2).

10. Marteau chirurgical selon une des revendications 1 à 3, **caractérisé en ce que** le canal d'introduction (7) est disposé dans un angle (β) approximativement d'environ 20 à 70°, en particulier d'environ 40 à 50°, par rapport à l'axe longitudinal (11) de la tête du marteau (2).

11. Marteau chirurgical selon la revendication 3 ou 10, **caractérisé en ce que** le canal d'introduction (7) et l'espace de verrouillage (9) sont disposés dans un angle (α) approximativement d'environ 20 à 70°, en particulier d'environ 30 à 40°, l'un par rapport à l'autre.

12. Marteau chirurgical selon une des revendications 1, 2, 3, 10 ou 11, **caractérisé en ce que** l'espace de verrouillage (9) est limité par au moins une, de préférence par plusieurs saillies (21), ces saillies étant en particulier disposées décalées les unes par rapport aux autres.

13. Marteau chirurgical selon une des revendications 1, 2, 3, 10, 11 ou 12, **caractérisé en ce que** l'espace de verrouillage (9) est en forme de trou oblong.

14. Marteau chirurgical selon une des revendications précédentes, **caractérisé en ce que** le sens de percussion du marteau (1), lorsqu'un dispositif (17) pour l'insertion et/ou l'extraction y est introduit, est essentiellement perpendiculaire à l'axe longitudinal (11) de la tête du marteau, la tête du marteau (2) pouvant tourner pendant le mouvement de percussion, de préférence autour de son axe longitudinal (11).

15. Marteau chirurgical selon une des revendications précédentes, **caractérisé en ce qu'**un manche de marteau (3) est fixé sur la tête du marteau (2).

16. Marteau chirurgical selon une des revendications précédentes, **caractérisé en ce que** l'évidement (6) est conçu de telle manière que le centre de gravité du marteau (1) se trouve approximativement dans le prolongement de l'axe longitudinal (12) du manche du marteau (3).

17. Marteau chirurgical selon une des revendications 15 ou 16, **caractérisé en ce que** le manche du marteau (3) est disposé de manière excentrée sur la tête du marteau (2).

18. Marteau chirurgical selon une des revendications précédentes, **caractérisé en ce que** des éléments de verrouillage supplémentaires, comme par exemple des broches de pression à rotule ou des dispositifs semblables, sont prévus dans l'espace de verrouillage (9) de la tête du marteau (2) pour assurer un blocage supplémentaire sur un dispositif (17) pour insérer et/ou extraire.

19. Marteau chirurgical selon une des revendications 15 à 18, **caractérisé en ce que** le manche du marteau (3) comporte une poignée (10), en particulier une poignée qui est aplatie sur au moins un côté.
